(19) Europäisches Patentamt
European Patent Office
Office européen des brevets

(11) Publication number: **0 150 107**
**A2**

(12) **EUROPEAN PATENT APPLICATION**

(21) Application number: 85300331.7

(22) Date of filing: 17.01.85

(51) Int. Cl.⁴: C 07 C 5/32
B 01 J 29/04

(30) Priority: 24.01.84 GB 8401834

(43) Date of publication of application:
31.07.85 Bulletin 85/31

(84) Designated Contracting States:
BE DE FR GB IT NL SE

(71) Applicant: The British Petroleum Company p.l.c.
Britannic House Moor Lane
London EC2Y 9BU(GB)

(72) Inventor: Barri, Sami Ali Ibrahim The British Petr. Comp.plc
Chertsey Road Sunbury-on-Thames
Middlesex TW16 7LN(GB)

(72) Inventor: Dave, Dilip The British Petroleum Comp. p.l.c.
Chertsey Road Sunbury-on-Thames
Middlesex TW16 7LN(GB)

(72) Inventor: Young, Dennis The British Petroleum Comp. p.l.c.
Chertsey Road Sunbury-on-Thames
Middlesex TW16 7LN(GB)

(74) Representative: Krishnan, Suryanarayana Kalyana et al,
BP INTERNATIONAL LIMITED Patents Division Chertsey Road
Sunbury-on-Thames Middlesex TW16 7LN(GB)

(54) **Production of unsaturated hydrocarbons by dehydrogenation.**

(57) This invention relates to a process for the production of unsaturated compounds by dehydrogenation of a corresponding saturated compound in the fluid phase in the presence of a supported gallium compound as catalyst. The support is a tectometallosilicate which has the following composition in terms of the mole ratios of the oxides:

$$(0.9 \pm 0.25)M_{\frac{4-m}{n}} : XO_2 : xSiO_2 : yH_2O:zQ$$

and is suitably the zeolite Theta-1. The catalyst composition may be subjected to a thermochemical treatment prior to contact with the saturated compound to be dehydrogenated.

EP 0 150 107 A2

Croydon Printing Company Ltd.

PRODUCTION OF UNSATURATED HYDROCARBONS BY DEHYDROGENATION

The present invention relates to a process for the production of unsaturated hydrocarbons e.g. olefins from the corresponding saturated compounds by dehydrogenation in the presence of tectometallosilicates, such as zeolites.

Zeolites are well known natural and synthetic compositions. Many of them have been demonstrated to have catalytic properties for various types of hydrocarbon conversion and related reactions. Zeolites can be defined as ordered porous crystalline aluminosilicates having a framework structure consisting of a rigid regular three dimensional network of $SiO_4$ and $AlO_4$ tetrahedra in which the tetrahedra are cross-linked by sharing the oxygen atoms and which are sufficiently open to accommodate at least water molecules. Such structures generally contain a regular array of small voids interconnected by channels or pores. The dimensions of the voids and channels can range from those of water to those of quite large molecules. For a given framework structure, the dimensions of the voids and channels are limited to a small number of values, which can vary from structure to structure. Thus these structures are capable of absorbing molecules of certain dimensions while rejecting those of dimensions larger than a critical value which varies with structure. This has led to zeolites being used as molecular sieves. Zeolites belong to a class of materials that can be termed tectoaluminosilicates which comprise (in addition to zeolites) felspars and felspathoids. All oxygen atoms forming the tetrahedra are shared, thus the ratio of total aluminium and silicon atoms to

oxygen atoms is 1:2. The inclusion of aluminium in the framework leads to a net negative charge which is balanced by the inclusion in the crystal of an electrochemical equivalence of cations, for example alkali metal, alkaline earth metal, hydrogen or ammonium cations or mixtures thereof. This can be expressed by a formula in which the ratio of Al to the number of the various cations such as Ca/2, Sr/2, Na, K, Li or generally M/n (where M is the cation and n is the formal oxidation state of the cation) is equal to unity. Additionally in zeolites, but not in felspars and some felspathoids, the framework is sufficiently open to accommodate water molecules as well as cations. This enables these cations to be exchanged in their entirety or partially by other cations using ion-exchange techniques in a conventional manner. These materials can exhibit specific affinities for specific cations and can thus be used as selective ion-exchangers. By means of ion-exchange, it is possible to vary the size of the pores in a given crystalline zeolite material, modifying its molecular sieve properties. Also by means of ion-exchange the catalytic properties of these materials can be altered. In addition to the framework and charge-compensating cations, zeolites can contain other materials such as water and organic molecules, (hydrated) salts and oxides of e.g. Na, Al and Si introduced during synthesis, or formed or added during subsequent treatments. Zeolites are best characterised according to framework structure type, i.e. on the topology of the framework, irrespective of composition, distribution of different tetrahedral atoms, cell dimensions and symmetry. A code consisting of three capital letters has been adopted for each known structure type following the recommendations by IUPAC on zeolite nomenclature ("Chemical Nomenclature, and Formulation of compositions, of Synthetic and Natural Zeolites", IUPAC yellow booklet, 1978) and a compilation of 38 known zeolite structure types has been published by The Structure Commission of the International Zeolite Association ("Atlas of Zeolite Structure Types", by Meier, W.M. and Olsen, D.H. (1978), distributed by Polycrystal Book Service, Pittsburgh, Pa, USA). In addition to the groups classified by known structure type, there is

a further group of crystalline zeolite materials whose X-ray diffraction patterns, sorption, ion-exchange and related properties indicate that they do not have known structure types but appear to have new, as yet undetermined structure types. An example of such a material is the novel porous crystalline aluminosilicate designated Theta-1 and described in our published European patent specification No. 0057049.

Zeolites (and other tectoaluminosilicates) belong to a larger class of materials that can be termed tectometallosilicates which can be defined in the same way as tectoaluminosilicates except that the aluminium is replaced by a range of elements, which includes, it is claimed, Ti, Zr, V, Cr, Mo, Mn, Fe, Co, Rh, Ni, Zn, B, Al, Ga, Ge, Sn, As and Sb, but in some cases the claimed materials have not been well characterised. In some cases (where the element has the same formal oxidation state as Si i.e. +4) the resultant framework is electroneutral and the resultant materials resemble crystalline silicas. In other cases there is a resultant framework negative charge which must be compensated by cations as in tectoaluminosilicates. In some cases the materials have porous frameworks like those of zeolites or porous crystalline silicas which they therefore resemble.

There are several prior art publications which indicate that feedstocks such as paraffins, especially $C_3$-$C_8$ paraffins can be dehydrogenated to olefins. Of these GB 1507778, GB 1507549 and GB 1537780 all in the name of the British Petroleum Company p.l.c. refer to the use of gallium oxide on a silica or alumina support as a dehydrogenation catalyst. However, the selectivity of gallium oxide on silica for olefins is only viable at relatively low conversions, the selectivity dropping in favour of aromatics formation and (hydro-) cracking reactions at high conversions. In addition these catalysts suffer from coke deposition and deactivate rapidly.

Another class of catalysts are the types disclosed in our EP-A-0042252 which refers to the use of catalysts containing a gallium compound on a low acid support for the dehydroisomerisation

of paraffins e.g. n-butane to isobutene.

In our patent publications GB 1561590 and EP 0024930, gallium compounds supported on zeolites of the MFI, MEL and FER types, ZSM-12, ZSM-8 and zeolite-beta are said to catalyse such dehydrogenation reactions although the products of the reaction are primarily aromatics combined with some cracked products.

A further publication, EP-A-0036292, describes the use of aluminosilicate zeolites for conversion of paraffins to aromatics and (hydro-) cracked products.

Generally when producing olefins from paraffins over MFI-type aluminosilicate catalysts, it is the formation of aromatics that has to be suppressed by modification of the MFI-type aluminosilicate.

Theta-1 type aluminosilicates, as described in our European Patent Specification No. 0057049 are expected to have similar catalytic properties to MFI-type aluminosilicates because of very similar physicochemical properties. MFI- and Theta-1 type aluminosilicates can be prepared with very similar $SiO_2/Al_2O_3$ ratios and with near identical shape-selective sorption characteristics. For example, they both adsorb and desorb xylenes. They both can be prepared from very similar starting gels (including the same template such as diethanolamine) subjected to identical digestion parameters, see for example our European Patent Specification No. 0057049. Thus it could be reasonably expected that conversion of paraffins over a catalyst containing a gallium compound on an un-modified hydrogen-form of Theta-1 aluminosilicate should lead to aromatics and paraffins if exposed to conditions under which a catalyst containing a gallium compound on an unmodified hydrogen-form of the MFI type aluminosilicate leads to formation of aromatics and paraffins.

Surprisingly, it has now been found that if a gallium compound supported on a Theta-1 type aluminosilicate or on another tectometallosilicate of similar structure is used for the conversion of paraffins, the process is highly selective towards unsaturated aliphatics. In addition the catalyst life is long relative to gallium loaded alumina or silica catalysts.

Accordingly, the present invention is a process for the production of unsaturated compounds by dehydrogenation of a corresponding saturated compound in the fluid phase in the presence of a supported gallium compound as catalyst, characterised in that the support is a tectometallosilicate which has the following composition in terms of the mole ratios of the oxides:

$$(0.9 \pm 0.25)M_{\frac{4-m}{n}} : XO_2 : xSiO_2 : yH_2O:zQ$$

wherein M is at least one cation having a valence n, X is one or more of the metals selected from Al, Ga, Zn, Fe, Cr and B, m is the valency of the metal X in the metal oxide $XO_2$, Q is a template used in the synthesis of the tectometallosilicate, x is at least 10, y/x is from 0 to 5, and z/x is 0-20, and the tectometallosilicate in its organo-free hydrogen-form has an X-ray diffraction pattern substantially as set forth in Table A of this specification.

Thus the tectometallosilicates used in the present invention have a Theta-1-type structure as described in our European Patent Specification No. 0057049.

It is preferred that the Theta-1 type tectometallosilicate is in the organic-free hydrogen form in the catalyst.

By the term "organic-free hydrogen-form" is meant here and throughout this specification that the tectometallosilicate has been rendered substantially free of organic material introduced during synthesis or subsequent treatments, and the cation M is hydrogen. The tectometallosilicates prepared from organic bases contain organics as synthesised and these can suitably be removed by calcination in air. The tectometallosilicates prepared from ammonia do not contain organics as synthesised. Therefore, in such a case, an organics removal step such as calcination in air is not essential.

The $H_2O$ content "y" of the tectometallosilicate is the water of hydration and will depend, within the ratios set out above, upon the conditions under which it is dried, calcined, subjected to further aqueous treatments or combinations thereof after synthesis. The $H_2O$ content "y" does not include water which may be notionally present when the cation M is hydrogen.

The 'template' is a chemical agent, e.g. an organic or inorganic base which encourages crystallisation to proceed towards a particular framework structure or structures.

The content "z" of template "Q" in the tectometallosilicate will also depend upon the conditions under which the tectometallosilicate is washed, calcined or subjected to further aqueous treatments or combinations thereof after synthesis, and also on the synthesis parameters of the gallosilicate, particularly the proportion of Q present in the original hydrogel. The molar ratio of the template Q to silica, i.e. z/x is preferably 0-5 in the tectometallosilicate as synthesised. The template content is usually highest for the "parent" tectometallosilicate. Complete removal of the template, if present, is usually only possible by thermal or oxidative degradation or both.

By the "parent" tectometallosilicate is meant throughout this specification the product of synthesis and washing and optionally drying.

The cation M in the tectometallosilicate may be selected from $H^+$, ammonium, alkali metal cations, alkaline earth metal cations, organic nitrogen containing cations, aluminium cation, gallium cation and mixtures thereof.

The cations present in the tectometallosilicate may be replaced using conventional ion exchange techniques either wholly or partially by other cations e.g. hydrogen ions or metal cations.

The organic-free hydrogen-form of the tectometallosilicate may be produced by known methods such as exchange with hydrogen ions or with ammonium cations followed by one or more calcinations or a combination of the two followed by one or more calcination stages, if the tectometallosilicate still contained ammonium ions.

The metal X in the metal oxide $XO_2$ is preferably Al, Ga, Zn, Fe, B or Cr. In the tectometallosilicate the molar ratio of silica to metal X is preferably from 20:1 to 500:1.

The tectometallosilicates according to the present invention have in their organic-free hydrogen form an X-ray diffraction pattern shown in Table 1 below. The specific values in the Table

0150107

were determined using copper K-alpha radiation and a computer step scan.

The peak heights, I, and their position as a function of 2-theta, where theta is the Bragg angle, were read from the spectrometer output. From this output the relative intensities 100 x $I/I_o$, where $I_o$ is the intensity of the strongest peak, and d the interplanar spacing in A, corresponding to the recorded peaks were calculated.

It will be understood by those skilled in the art that the X-ray diffration pattern of tectometallosilicates may vary in the values of $I/I_o$ and the d-spacing depending for example upon whether the sample being examined is calcined or uncalcined, upon the temperature of calcination, upon the nature of the cation present in the tectometallosilicate, the mole ratio of silica to metal oxide, and the particle size of the tectometallosilicate.

The tectometallosilicate is suitably produced by mixing a source of silica, a source of gallia, a source of alkali metal(s), water and an organic or inorganic nitrogen containing base until a homogeneous gel is formed and crystallising the gel at a temperature above 70°C.

TABLE 1

| 2-theta (degrees) | d-spacing (Angstroms) | Relative Intensity 100 x $I/I_o$ |
|---|---|---|
| 8.06 ± 0.2 | 11.25 - 10.70 | 50 to 100 |
| 10.06 ± 0.2 | 9.01 - 8.63 | 5 to 30 |
| 12.69 ± 0.2 | 7.09 - 6.87 | 10 to 30 |
| 16.28 ± 0.2 | 5.51 - 5.38 | 5 to 15 |
| 19.40 ± 0.2 | 4.62 - 4.53 | 5 to 15 |
| 20.26 ± 0.2 | 4.43 - 4.34 | 50 to 100 |
| 24.11 ± 0.2 | 3.72 - 3.66 | 50 to 100 |
| 24.52 ± 0.2 | 3.66 - 3.60 | 30 to 90 |
| 25.65 ± 0.2 | 3.50 - 3.45 | 15 to 45 |

scanned up to 2 theta = 32

The gallium in the catalyst composition may be present as gallium oxide and/or as gallium ions if cations in the tectometallosilicate support have been exchanged with gallium ions. In the case where the cations in the tectometallosilicate have been exchanged for gallium ions, the gallium ion is suitably provided as an aqueous solution of a gallium salt such as for instance gallium nitrate, gallium chloride or gallium sulphate. Such catalysts may be produced by conventional ion exchange techniques and the catalysts so produced are subsequently dried. For example and aqueous solution of a gallium compound such as gallium nitrate may be placed in contact with the tectometallosilicate at ambient or elevated temperature, e.g. by refluxing. The exchanged tectometallosilicate is then separated by decantation followed by filtration, washed several times with deionised water and finally dried. Before addition to the aqueous solution of the gallium compound, the tectometallosilicate may be treated in various ways e.g. as described in our published copending European Patent Application No. 0024930.

The present invention may also be carried out using catalysts in which the gallium deposited is impregnated on the surface of the tectometallosilicate or is incorporated in the intra-crystalline zeolite cavities as a gallium compound which gives rise to gallium oxide during activation of the catalyst prior to contact with the hydrocarbon feedstock. An example of a suitable gallium compound is gallium nitrate. Conventional impregnation techniques may be used to produce these catalysts.

The impregnation may be achieved by preparing a solution, suitably in aqueous solution, of a gallium compound such as for example gallium nitrate and adding the tectometallosilicate to this aqueous solution with thorough stirring to form a paste. The paste is subsequently dried at an elevated temperature under vacuum.

Where the catalyst composition is prepared by using a compound of gallium which ionises in aqueous solution, for example gallium nitrate, it is inevitable that some of the gallium ions will be exchanged with the cations in the tectometallosilicate even if the preparation was by impregnation of the tectometallosilicate.

The Theta-1 type tectometallosilicates used in the present invention may be bound in a suitable binding material before or after loading with gallium using methods and binding materials well known in the art. The catalyst composition may be subjected to further treatments e.g. thermochemically by heating for instance in an oxidative environment such as dry air or in a reductive environment such as dry hydrogen. Such further treatments may be carried out during one or more of the catalyst preparation stages.

It is preferable to carry out the thermochemical treatment in hydrogen especially after the gallium loading stage. For instance, such treatment with hydrogen may be carried out using a carrier gas which contains 1-100% v/v, preferably from 30-100% v/v of hydrogen. The treatment temperature is suitably from 450-700°C, preferably from 525-650°C. The treatment pressure may vary from 1 to 10 bar absolute but is preferably 1 bar absolute. The treatment may be carried out over a duration of 5 minutes to 200 hours, preferably from 1-20 hours.

A particularly useful treatment is to treat the bound gallium-loaded catalyst with steam to improve the catalyst life and selectivity to olefins. The steam treatment is suitably carried out with a carrier gas containing 1-100% v/v, preferably from 10-100% v/v of steam at a pressure from 0.1 to 10 bar absolute, preferably at 1 bar absolute and a temperature from 300-750°C, preferably from 500-700°C. The duration of the steam treatment may vary from 5 minutes to 200 hours but is preferably from 1 to 12 hours.

It will be appreciated by those skilled in the art that the conditions specified for any of the aforementioned treatments will be interdependent. Increasing the severity of one or more of the parameters may allow a reduction in the severity of the other relevant parameters. In the case of steam treatment for instance raising the treatment temperature can be expected to shorten the duration of the treatment.

The saturated compounds which may be dehydrogenated to the corresponding unsaturated compounds by contact with the supported catalysts as herein defined are preferably saturated $C_2$-$C_{20}$ hydrocarbons, most preferably $C_2$-$C_5$ paraffins and mixtures thereof.

The feed may be diluted for instance with hydrogen or steam.

The dehydrogenation reaction is suitably carried out from 250-800°C, the precise temperature depending upon other variables such as feedstock and reaction conditions. For instance ethane is converted to ethylene only at temperatures above 650°C and 1 bar pressure whereas furan can be formed from tetrahydrofuran at about 300°C and 1 bar pressure.

The dehydrogenation reaction may be carried out at reduced or elevated pressure, suitably from 0.1-20 bar pressure, preferably from 0.5-10 bar pressure and most preferably from 1-5 bar pressure.

The space velocity of the feedstock over the gallium loaded tectometallosilicate is suitably in the range of 0.5-1000 WHSV, preferably 0.5-20 WHSV.

The products of the dehydrogenation reaction are principally hydrocarbons which are rich in light olefins e.g. ethylene, propylene and butylenes. The dehydrogenation reaction is efficient and selective in that by-products such as methane, carbon monoxide and ethane are formed only in very small amounts.

The light olefins such as the $C_2$-$C_4$ olefins are valuable petrochemical feedstock for producing polyolefins, alcohols, surfactants and alkylaromatics, and as a feedstock for producing diesel and jet fuels.

The process can be combined with an olefin to hydrocarbon liquids process to give a combined paraffins to hydrocarbon liquids process.

The present invention is further illustrated with reference to the following Examples.

EXAMPLES

A. Catalyst Preparation

(i) A solution was prepared from a mixture of sodium aluminate (12.6g), sodium hydroxide (6.65g) and water (140g). Diethanolamine (DEA, 180g) was melted and added to the solution and the resultant solution "X" was stirred and maintained at room temperature. 500g of a commercial silica sol., 'Ludox AS40' (Reg. Trade Mark) which contains 40% by weight of silica, was further diluted with (354g) water to form solution "Y". Thereafter solution Y was added

dropwise to solution X over a period of 15 minutes with vigorous stirring. Stirring was continued for a further 20 minutes and then the resultant gel which had the composition:

2.64 $Na_2O$ : 31.8 (DEA) : $Al_2O_3$ : 61.9 $SiO_2$ : 819 $H_2O$

was transferred to an oven and crystallised with agitation at 175°C for 48 hours in a revolving stainless steel pressure vessel. The product was filtered, washed and dried at 90°C.

(ii) The dried product was calcined by transferring to an oven and heating to 550°C over 4 hours, maintaining at 550°C for 60 hours and then cooling. The calcined product was refluxed in 10 volume per cent aqueous nitric acid for 2.5 hours, filtered, washed and refluxed in 0.67 M aqueous ammonium nitrate solution for 4 hours. In both cases the solution: zeolite weight ratio was 6:1. The product was filtered, washed, dried at 90°C, transferred to an oven and heated to 550°C over 4 hours, maintained at 550°C for 16 hours then cooled. The X-ray powder diffraction pattern of the resultant material is shown in Table 1 and shows the product to be crystalline Theta-1. The crystalline powder was refluxed in an aqueous gallium nitrate solution as described in Example 1 of European Patent Application No. 80302997.4. The resultant material was mixed with twice its weight of Ludox AS40 and just sufficient distilled water to wet the mixture. The resulting slurry was dried at 90°C and the resultant cake was broken and sieved to pass 12 to 30 mesh (BSS). The granules so prepared were steamed (20% v/v steam in air) at 550°C for 2 hours.

(iii) The zeolite was prepared as described in section A(i) and was converted to the sodium form by refluxing with 1M aqueous sodium nitrate solution (1g zeolite:6 ml solution). This operation was carried out twice. The solid was filtered, washed with distilled water and dried at 90°C. The dried zeolite was then calcined at 550°C to remove organic material as described in section A(ii).

The pH of an aqueous gallium nitrate solution (1g gallium/litre solution) was adjusted to 4 with tetraethylammonium hydroxide solution. The zeolite was added to this solution (1g of zeolite/6 ml solution) and the mixture refluxed for 4 hours. The

zeolite was filtered, washed and dried at 90°C, and then formed into tablets which were broken and sieved to pass 12 to 30 mesh (BSS).

B. Dehydrogenation Reaction

6 ml samples of the catalysts prepared as in sections A(ii) or A(iii) above were loaded into a reactor and activated in flowing air at 550°C for 4 hours. The air was flushed out with dry nitrogen and then n-butane was passed over the catalyst in each case. The reaction conditions used and the results obtained are given in Table 2.

Table 2

Dehydrogenation of Butane over Theta-1

| Catalyst | A1 | A2 | A1 | A2 | A2 | A3 |
|---|---|---|---|---|---|---|
| Temperature °C | 530 | 530 | 600 | 590 | 600 | 570 |
| WHSV (1) $h^{-1}$ | 4.5 | 4.8 | 4.7 | 4.7 | 0.8 | 5.8 |
| Butane conversion (2) | 24 | 17 | 26 | 24 | 50 | 24 |
| Selectivity to butenes (3) w/w% | 4 | 16 | 30 | 40 | 34 | 55 |
| Selectivity to olefins (4) w/w% | 30 | 42 | 50 | 67 | 63 | 83 |

A1 was prepared as described in section A(ii) but not steam-treated

A2 was prepared as described in section A(ii)

A3 was prepared as described in Section A(iii) and steam-treated

(1) WHSV = Weight hourly space velocity i.e. weight of butane fed per weight of catalyst per hour.

(2) Butane conversion = 100 % weight of butane in the product

(3) Selectivity to butenes = $\frac{\text{\% weight of butene produced}}{\text{Butane conversion (1)}} \times 100$

(4) Selectivity to olefins = $\frac{\text{\% weight of olefins produced}}{\text{Butane conversion (1)}} \times 100$

Claims:

1. A process for the production of unsaturated compounds by dehydrogenation of a corresponding saturated compound in the fluid phase in the presence of a supported gallium compound as catalyst, characterised in that the support is a tectometallosilicate which has the following composition in terms of the mole ratios of the oxides:

$$(0.9 \pm 0.25)M_{\frac{4-m}{n}} : XO_2 : xSiO_2 : yH_2O:zQ$$

wherein M is at least one cation having a valence n, X is one or more of the metals selected from Al, Ga, Zn, Fe, Cr and B, m is the valency of the metal X in the metal oxide $XO_2$, Q is a template used in the synthesis of the tectometallosilicate, x is at least 10, y/x is from 0 to 5, and z/x is 0-20, and the tectometallosilicate in its organo-free hydrogen-form has an X-ray diffraction pattern substantially as set forth in Table 1 of this specification.

2. A process according to claim 1 wherein the saturated compounds which may be dehydrogenated to the corresponding unsaturated compounds by contact with the supported catalyst is a saturated $C_2$-$C_{20}$ hydrocarbon.

3. A process according to any one of the preceding claims wherein the catalyst composition is subjected to a thermochemical treatment by heating in an oxidative or in a reductive environment during one or more of the catalyst preparation stages.

4. A process according to claim 3 wherein the thermochemical treatment is carried out in hydrogen after the gallium loading stage.

5. A process according to claim 4 wherein the treatment with hydrogen is carried out using a carrier gas which contains 1-100% v/v, at a temperature from 450-700°C and at a pressure from 1 to 10 bar absolute.

6. A process according to claim 3 wherein the treatment is carried out on a bound gallium loaded catalyst with steam.

7. A process according to claim 4 wherein the steam treatment is carried out with a carrier gas containing 1-100% v/v of steam at a pressure from 0.1 to 10 bar absolute and at a temperature from 300-750°C.

8. A process according to any one of the preceding claims wherein the dehydrogenation reaction is carried out at a temperature from 250-800°C and a pressure from 0.1-20 bar at a WHSV of 0.5-1000.

9. A process according to any one of the preceding claims as hereinbefore described with reference to the Examples.